# EUROPEAN PATENT SPECIFICATION

(11) **EP 2 950 657 B1**
(45) Date of publication and mention of the grant of the patent: **11.03.2020**
(21) Application number: 14708943.7
(22) Date of filing: 29.01.2014
(51) Int. Cl.: A01N 25/08, A01N 63/04, C12N 9/42

(54) **NOVEL CHITIN/ DEMINERALISED DEHYDRATED CHITINACEOUS / CRUSTACEAN EXOSKELETON -BASED FORMULATION CONTAINING MICROBES THAT GENERATE CHITINASE/ PROTEASE ENZYMES**
NEUARTIGE, AUF CHITIN-/DEMINERALISIERTEM DEHYDRIERTEM CHITINÖSEM/KRUSTENARTIGEN EXOSKELETT BASIERENDE FORMULIERUNG MIT MIKROBEN ZUR ERZEUGUNG VON CHITINASE/PROTEASE-ENZYMEN
NOUVELLE PRÉPARATION À BASE D'EXOSQUELETTE DE CHITINE OU DE CRUSTACÉS/CHITINE DÉSHYDRATÉE(S) DÉMINÉRALISÉE(S) CONTENANT DES MICROBES PRODUISANT DES CHITINASES/PROTÉASES

(30) Priority: 30.01.2013 IN 414CH2013
(43) Date of publication of application: 09.12.2015
(73) Proprietor: Pelican Biotech&Chemical Labs Pvt. Ltd., Alappuzha District, Kerala 688 536 (IN)
(72) Inventor: NARAYANAN, Manoj, Chuzhattil, Alappuzha Kerala 688 536 (IN); RAO, Priya, Raghavendra, Alappuzha Kerala 688 536 (IN); JACOB, Thomas, Kottayam 686 004 Kerala (IN)
(74) Representative: Wynne-Jones IP Limited
(86) International application number: PCT/IB2014/058629
(87) International publication number: WO 2014/118707

(56) References cited:
- WO-A1-02/14540
- WO-A1-99/42594
- WO-A1-2006/089388
- US-A- 4 536 207
- US-A- 5 811 095
- D GIRIDHAR ET AL: "PURIFICATION, CHARACTERIZATION AND ANTIFUNGAL ACTIVITY OF CHITINASE FROM TRICHODERMA VIRIDE N9", JOURNAL OF CELL AND TISSUE RESEARCH, vol. 12, 1 January 2012 (2012-01-01), pages 3187-3192, XP055116564,
- CRISPINUS A. OMUMASABA ET AL: "Purification and characterization of a chitinase from Trichoderma viride.", THE JOURNAL OF GENERAL AND APPLIED MICROBIOLOGY, vol. 47, no. 2, 1 April 2001 (2001-04-01), pages 53-61, XP055116648, ISSN: 0022-1260, DOI: 10.2323/jgam.47.53
- PEREZ-MARTINEZ ET AL: "Overexpression, purification and characterization of the Trichoderma atroviride endochitinase, Ech42, in Pichia pastoris", PROTEIN EXPRESSION AND PURIFICATION, ACADEMIC PRESS, SAN DIEGO, CA, vol. 55, no. 1, 18 August 2007 (2007-08-18), pages 183-188, XP022206990, ISSN: 1046-5928, DOI: 10.1016/J.PEP.2007.05.009
- FAN ET AL: "Expression of a Beauveria bassiana chitinase (Bbchit1) in Escherichia coli and Pichia pastoris", PROTEIN EXPRESSION AND PURIFICATION, ACADEMIC PRESS, SAN DIEGO, CA, vol. 56, no. 1, 3 October 2007 (2007-10-03), pages 93-99, XP022284718, ISSN: 1046-5928, DOI: 10.1016/J.PEP.2007.06.012
- W. FANG ET AL: "Cloning of Beauveria bassiana Chitinase Gene Bbchit1 and Its Application To Improve Fungal Strain Virulence", APPLIED AND ENVIRONMENTAL MICROBIOLOGY, vol. 71, no. 1, 1 January 2005 (2005-01-01), pages 363-370, XP055116509, ISSN: 0099-2240, DOI: 10.1128/AEM.71.1.363-370.2005
- FAHEEM AMIN ET AL: "POTENTIAL OF TRICHODERMA SPECIES AS BIOCONTROL AGENTS OF SOIL BORNE FUNGAL PROPAGULES", JOURNAL OF PHYTOLOGY AN OPEN ACCESS JOURNAL, vol. 2, 1 January 2010 (2010-01-01), pages 38-41, XP055116569,
- S. B. CHAVAN ET AL: "Chitinolytic enzymes: An appraisal as a product of commercial potential", BIOTECHNOLOGY PROGRESS, vol. 29, no. 4, 18 July 2013 (2013-07-18), pages 833-846, XP055116703, ISSN: 8756-7938, DOI: 10.1002/btpr.1732
- V. Jegathambi ET AL: "Effect of Trichoderma sp. on Sclerotium rolfsii, the Causative Agent of Collar Rot on Zamioculcas zamiifolia and an on Farm Method to Mass Produce Trichoderma species", PLANT PATHOLOGY JOURNAL, vol. 9, no. 2, 1 February 2010 (2010-02-01), pages 47-55, XP055428118, PK ISSN: 1812-5387, DOI: 10.3923/ppj.2010.47.55

## Description

### FIELD OF INVENTION

The invention is a new product comprising of biopolymer chitin or demineralised dehydrated chitinaceous / crustacean exoskeleton and microbes that produce chitinase / protease enzymes.

The invention is a new product where the chitin or demineralised dehydrated chitinaceous / crustacean exoskeleton acts as a substrate for the microbes and also induces the production of chitinase / protease enzymes. The enzymes will act on the chitinaceous exoskeleton of pathogenic insects and fungus providing pesticidal activity. Chitin or demineralised dehydrated chitinaceous / crustacean exoskeleton populated with microbes are used as a bio-control agent that can be used in all agricultural applications.

This invention also brings out a novel product which comprises of chitin or demineralised dehydrated chitinaceous / crustacean exoskeleton with chitinase / protease enzymes along with microbes that produce chitnase/protease enzymes.

### BACKGROUND OF INVENTION

The invention brings out a novel product comprising of chitin or demineralised dehydrated chitinaceous / crustacean exoskeleton with microbes that produce chitinase / protease enzymes.

The prior art is use of chitin as a good agricultural soil/ root product. The prior art uses chitin as a soil ingredient which may induce chitinase / protease enzymes production in field condition. The limitation of the prior art is that additional microbes need to be added to induce chitinase which may not be acclimatised in the new environment. The present invention overcomes this limitation by the use of chitin or demineralised dehydrated chitinaceous / crustacean exoskeleton populated with microbes that produce chitinase / protease enzymes. There are no prior art on microbes populated in chitin or demineralised dehydrated chitinaceous / crustacean exoskeleton.

Another prior art is to use charcoal / talc / liquid broth as substrate to inoculate and supply biopesticidal microbes. The disadvantage of the prior art is that additional chitinaceous matrix that helps in in-situ chitinase enzyme generation has to be added as a separate component. The present invention proposes a product in which the microbes are allowed to grow in chitin/ demineralised dehydrated chitinaceous / crustacean exoskeleton which helps in chitinase / protease enzymes generation thereby populating both the enzymes as well as the microbes progressively unlike as in charcoal/talc where they remain dormant.

The prior art of using biopesticidal fungi like *Trichoderma viride* and *Beauveria bassiana* involves providing as inocula in a solid or liquid substrate. The disadvantage of the prior art is that the microbes are not active in the inert un-reactive matrix and does not produce any biopesticidal enzymes unless introduced in the field, creating delay in action. The present invention proposes a new product that not only contains inoculum but also contains chitinase / protease enzymes produced by the microbes in the chitnaceous substrate thereby providing instant effect. The microbes in the product as described in the present invention are active due to the presence of biopolymer chitin or demineralised dehydrated chitinaceous/ crustacean exoskeleton which provide the nutrients for microbial proliferation in the amorphous matrix resulting in enhanced insecticidal activity.

Prior art includes D GIRIDHAR ET AL, "Purification, Characterization and Antifungal Activity of Chitinase from Trichoderma Viride N9," Journal of Cell and Tissue Research, vol.12, 1 January 2012, pages 3187-3192, XP055116564. ABSTRACT: "A chitinase produced by *Trichoderma viride* N9 isolated from a soil sample collected from Nallamala forest, India, was purified and characterized. The enzyme was purified in three step procedure involving ammonium sulphate precipitation, sephadex G-100 gel filtration and DEAE-cellulose ion-exchange chromatography. Through the process 6-fold increase in purity with a specific activity of 142.7 U/mg proteins was obtained. The molecular mass of the purified chitinase was found to be 46 kDa by SDS-PAGE. It was optimally active at pH of 4 and at 40°C. The enzyme was stable from pH 3 to 6, and up to 50°C. Among the metals that were tested, the Fe²⁺ , Hg²⁺ , Mn²⁺ andCo²⁺ completely inhibited the enzyme activity. The enzyme was less sensitive to Al³⁺, Ca²⁺, Cu²⁺, and Zn²⁺. The purified chitinase showed antifungal activity against phytopathogenic fungi."

Prior art further includes US 5 811 095 (WILLIAMSON JOHN B [US] ET AL) 22 September 1998. ABSTRACT: "An insecticidal composition and its method of preparation are presented. The composition comprises a basal broth, a chitinase broth and a chemical fungicide. The basal broth has proteins hydrolyzed by papain and pancreatin while the chitinase broth is prepared by fermenting chitin and a chitinase-producing bacterial culture. The insecticidal composition has synergistic activity."

Prior art further includes Jegathambigai, V., RS Wilson Wijeratnam, and R. L. C. Wijesundera, "Effect of Trichoderma sp. on Sclerotium rolfsii, the Causative Agent of Collar Rot on Zamioculcas zamiifolia and an on Farm Method to Mass Produce Trichoderma species," Plant Pathology Journal 9.2 (2010): 47-55. ABSTRACT: "The antagonistic effect of three local isolates of *Trichoderma viride* and one local isolate of *Trichoderma harzianum* were tested against the pathogenic fungus *Sclerotium rolfsii.* The latter organism is responsible for major loss due to collar rot of the ornamental crop *Zamioculcas zamiifolia* in Sri Lanka. The disease causes massive losses. The antagonistic potential of the local isolates against the phytopathogenic fungi *Sclerotium rolfsii* was investigated in dual culture, poison food technique, pot trials and field trials on *Zamioculcas zamiifolia* plants. All *Trichoderma* isolates tested under in-vitro conditions significantly inhibited the growth of *S*. *rolfsii.* Of these isolates, *Trichoderma viride* isolate Tv1, showed highest percentage inhibition and was thus selected for *in vivo* field trials. Data recorded from bi monthly field application of this organism over the two growing seasons, confirmed the success of the treatment in controlling collar rot disease at the economic threshold level. Field application of testing isolate *T. viride* Tv1 as a conidial suspension (10¹¹ cfu mL⁻¹) greatly reduced the disease incidence of *Zamioculcas zamiifolia* plants by a percentage of 75.54%. On farm mass production of this isolate was developed to help facilitate the establishment of an integrated eco-friendly disease management system for growers of *Zamioculcas zamiifolia.* Different media was also evaluated to mass produce the *Trichoderma* isolate. The media evaluated in this study included the solid substrates barley seeds, paddy, cow pea, maize and sorghum and semi solid, liquid substrates such as potato dextrose, rice extract, paddy extracts, respectively. Although mycelial growth was fastest in barley and paddy media. And the highest yield of spores of the *Trichoderma* isolate was observed 7 days after inoculation in Barley and Paddy media."

The present invention provides for a product where the microbes and the chitinase/protease enzymes are populated in demineralised dehydrated chitinaceous / crustacean exoskeleton or on chitin. These enzymes will degrade the exoskeleton of pathogenic insects and fungi providing pesticidal activities.

The present invention is set out in the appended claims. Any embodiments, aspects or examples of the present description/disclosure that do not fall within the scope of said claims are provided for illustrative purposes only and do not form part of the present invention.

### OBJECT OF THE PRESENT INVENTION

An object of this invention is to provide a completely organic, eco-friendly, non-toxic chitin- based bio-control agent for the in-situ generation of chitinase / protease enzymes for use against insects in the soil / roots of the plants. This product can be used in all agriculture applications.

Another object of this invention is to use chitin or demineralised dehydrated chitinaceous / crustacean exoskeleton as a matrix, with microbes producing chitinase / protease enzymes for use in the *in-situ* generation of chitinase / protease enzymes and thereby using it in bio-control applications in agriculture.

Yet another object of the invention is to provide a new product that not only contains inocula but also contains chitinase / protease enzymes produced by the microbes.

### DESCRIPTION OF THE INVENTION:

According to this invention, there is provided an amorphous matrix consisting of versatile biopolymer chitin or demineralized dehydrated chitinaceous / crustacean exoskeleton in combination with chitinase / protease enzymes- producing microbes including fungi like *Trichoderma viride* and *Beauveria bassiana,* to be used as biocontrol / biopesticidal agents for application in the field of agriculture.

Fresh chitinaceous/ crustacean exoskeleton is soaked in 5% hydrochloric acid for four hours to get demineralised(DM) chitinaceous/crustacean exoskeleton. The demineralised shell is then subjected to solvent extraction using acetone to obtain dehydrated demineralised shell (DM-DH) shell. The DM-DH shell, after acetone extraction, is subjected to deproteinization using 3-5% sodium hydroxide and then washed to neutral pH and dried to obtain chitin. Chitin or DM-DH shell can be used for the preparation of the product.

To 12 grams of potato dextrose broth, add 500 ml distilled water. Sterilize the media in an autoclave. Allow it to cool to room temperature. Innoculate the pure culture with microbes that produce chitinase /protease enzymes. Keep the inoculated media in a rotary shaker for 72 hours. Filter the broth and mix together. Pour the mixed broth into 1 kg of chitin/ DM-DH flakes. Air dry the sample with loss of drying not less than 10%.

The invention will now be illustrated with working examples.

In one embodiment, the invention provides a product consisting of:
a) demineralized and dehydrated chitinaceous exoskeleton; and
b) chitinase producing microbe selected from a group consisting of *Trichoderma viride* and *Beauveria bassiana,*
wherein the total microbial load of the product is not less than 10⁸ cfu/g (colony forming units/gram), the chitinase activity is not less than 1 U/gds (unity per gram dry substrate) and
said product is an air-dried mixture with a loss on drying not less than 15% of a mixture consisting of a dehydrated demineralized chitinaceous exoskeleton and chitinase producing *Trichoderma viride* or *Beauveria bassiana* containing broth.

In another embodiment, the invention provides a product wherein the demineralized and dehydrated chitinaceous exoskeleton is a crustacean exoskeleton.

In another embodiment, the invention provides a product wherein the crustacean exoskeleton is prawn exoskeleton.

In yet another embodiment, the invention provides a process for the preparation of the product, comprising the steps of:
a) demineralizing in 5% hydrochloric acid fresh chitinaceous exoskeleton for four hours;
b) extracting the demineralized chitinaceous exoskeleton with acetone to obtain dehydrated demineralized chitinaceous exoskeleton;
c) adding chitinase producing microbe containing broth to the product of step b), wherein the chitinase producing microbe is selected from a group consisting of *Trichoderma viride* and *Beauveria bassiana;* and
d) air-drying the mixture of step c), with the loss on drying not less than 15%.

In another embodiment, the invention provides a process wherein the chitinaceous exoskeleton is a crustacean exoskeleton.

In another embodiment, the invention provides a process wherein the crustacean exoskeleton is prawn exoskeleton.

In another embodiment, the product of the present invention can be used as a biocontrol agent.

It is to be understood that the specific example being given here by way of illustration and are not intended to be taken restrictively to imply any limitation on the scope of the present invention.
**Example 1:** Fresh prawn shell (15 kg) was demineralised using 5% Hydrochloricacid and then dehydrated to obtain dehydrated Demineralised (DM-DH) shell. This shell was subjected to deproteinization using 3-5 % sodium hydroxide. The shell obtained was washed to neutral pH and dried to obtain chitin. This was used as the matrix for the formulation. To 24 grams of potato dextrose broth added 1 liter of distilled water. Sterilized the media in an autoclave. Allowed it to cool to room temperature. Added the fungal inoculum of *Trichoderma viride* or *Beauveria bassiana* into the media. Kept in rotary shaker for 72 hours. Filtered the broth and poured 100 ml of the broth into 1 kg chitin flakes. Air-dried the mixture to LOD (loss on drying-moisture) content not less than 15-20% and gave an assay of Microbial load of 10⁸ cfu/gm and Chitinase enzyme activity: not less than 1U/gds
**Example 2:** Fresh prawn shell (15 kg) was demineralised using 5% Hydrochloricacid and then dehydrated to obtain dehydrated Demineralised (DM-DH) shell. This DM-DH shell was the matrix in the preparation of the present formulation. To 24 grams of potato dextrose broth added 1 liter of distilled water. Sterilized the media in an autoclave. Allowed it to cool to room temperature. Added the fungal inoculum of *Trichoderma viride* or *Beauveria bassiana* into the media. Kept p in rotary shaker for 72 hours. Filtered the broth. Poured 100 ml of broth into 1 kg of DM-DH flakes. Air-dried the mixture to loss on drying moisture content LOD not less than 15-20 % and gave an assay of Microbial load 10⁸ cfu / gm and Chitinase enzyme activity not less than 1U/gds

## Claims

1. A product consisting of:
a) demineralized and dehydrated chitinaceous exoskeleton; and
b) chitinase producing microbe selected from a group consisting of *Trichoderma viride* and *Beauveria bassiana,*
wherein the total microbial load of the product is not less than 10⁸ cfu/g (colony forming units/gram), the chitinase activity is not less than 1 U/gds (unity per gram dry substrate) and said product is an air-dried mixture with a loss on drying not less than 15% of a mixture consisting of a dehydrated demineralized chitinaceous exoskeleton and chitinase producing *Trichoderma viride* or *Beauveria bassiana* containing broth.

2. The product according to claim 1, wherein the demineralized and dehydrated chitinaceous exoskeleton is a crustacean exoskeleton.

3. The product according to claim 2, wherein the crustacean exoskeleton is prawn exoskeleton.

4. A process for the preparation of a product according to claim 1 , comprising:
a) demineralizing in 5% hydrochloric acid fresh chitinaceous exoskeleton for four hours;
b) extracting the demineralized chitinaceous exoskeleton with acetone to obtain dehydrated demineralized chitinaceous exoskeleton;
c) adding chitinase producing microbe containing broth to the product of step b), wherein the chitinase producing microbe is *Trichoderma viride* or *Beauveria bassiana;* and
d) air-drying the mixture of step c), with the loss on drying not less than 15%.

5. The process according to claim 4, wherein the chitinaceous exoskeleton is a crustacean exoskeleton.

6. The process according to claim 5, wherein the crustacean exoskeleton is prawn exoskeleton.

7. The product according to any one of claims 1 to 3 for use as a biocontrol agent.

## Patentansprüche

1. Produkt, bestehend aus:
a) entmineralisiertem und dehydriertem chitinhaltigem Exoskelett und
b) Chitinase produzierender Mikrobe, ausgewählt aus einer Gruppe bestehend aus *Trichoderma viride* und *Beauveria bassiana,*
wobei die mikrobielle Gesamtbelastung des Produkts nicht weniger als 10⁸ KBE/g (koloniebildende Einheiten/Gramm) beträgt, die Chitinase-Aktivität nicht weniger als 1 E/gTS (Einheit pro Gramm trockenes Substrat) beträgt und das Produkt ein luftgetrocknetes Gemisch mit einem Trocknungsverlust von nicht weniger als 15 % eines Gemischs ist, das aus einem dehydrierten entmineralisierten chitinhaltigen Exoskelett und einer Chitinase produzierenden, *Trichoderma viride* oder *Beauveria bassiana* enthaltenden Bouillon besteht.

2. Produkt nach Anspruch 1, wobei das entmineralisierte und dehydrierte chitinhaltige Exoskelett ein Krustazeen-Exoskelett ist.

3. Produkt nach Anspruch 2, wobei das Krustazeen-Exoskelett ein Garnelenexoskelett ist.

4. Verfahren zur Herstellung eines Produkts nach Anspruch 1, umfassend:
a) Entmineralisieren von frischem chitinhaltigem Exoskelett in 5%-iger Salzsäure für vier Stunden;
b) Extrahieren des entmineralisierten chitinhaltigen Exoskeletts mit Aceton, um dehydriertes entmineralisiertes chitinhaltiges Exoskelett zu erhalten;
c) Zugeben von Chitinase produzierende Mikrobe enthaltender Bouillon zu dem Produkt von Schritt b), wobei die Chitinase produzierende Mikrobe *Trichoderma viride* oder *Beauveria bassiana* ist; und
d) Lufttrocknen des Gemischs von Schritt c), wobei der Trocknungsverlust nicht weniger als 15 % ist.

5. Verfahren nach Anspruch 4, wobei das chitinhaltige Exoskelett ein Krustazeen-Exoskelett ist.

6. Verfahren nach Anspruch 5, wobei das Krustazeen-Exoskelett ein Garnelenexoskelett ist.

7. Produkt nach einem der Ansprüche 1 bis 3 zur Verwendung als ein biologisches Pflanzenschutzmittel.

## Revendications

1. Produit constitué par :
a) un exosquelette à base de chitine déminéralisé et déshydraté ; et
b) un microbe produisant de la chitinase choisi dans un groupe constitué par *Trichoderma viride* et *Beauveria bassiana,*
dans lequel la charge microbienne totale du produit n'est pas inférieure à 10⁸ ufc/g (unités formant colonie/gramme), l'activité chitinase n'est pas inférieure à 1 U/gds (unité par gramme de substrat sec) et ledit produit est un mélange séché à l'air avec une perte à la dessiccation non inférieure à 15 % d'un mélange constitué par un exosquelette à base de chitine déminéralisé et déshydraté et un bouillon contenant *Trichoderma viride* ou *Beauveria bassiana* produisant de la chitinase.

2. Produit selon la revendication 1, dans lequel l'exosquelette à base de chitine déminéralisé et déshydraté est un exosquelette de crustacé.

3. Produit selon la revendication 2, dans lequel l'exosquelette de crustacé est un exosquelette de crevette.

4. Procédé pour la préparation d'un produit selon la revendication 1, comprenant :
a) la déminéralisation dans de l'acide chlorhydrique à 5 % d'exosquelette à base de chitine frais pendant quatre heures ;
b) l'extraction de l'exosquelette à base de chitine déminéralisé avec de l'acétone pour obtenir un exosquelette à base de chitine déminéralisé et déshydraté ;
c) l'ajout d'un bouillon contenant un microbe produisant de la chitinase au produit de l'étape b), dans lequel le microbe produisant de la chitinase est *Trichoderma viride* ou *Beauveria bassiana* ; et
d) le séchage à l'air du mélange de l'étape c), avec une perte à la dessiccation non inférieure à 15 %.

5. Procédé selon la revendication 4, dans lequel l'exosquelette à base de chitine est un exosquelette de crustacé.

6. Procédé selon la revendication 5, dans lequel l'exosquelette de crustacé est un exosquelette de crevette.

7. Produit selon l'une des revendications 1 à 3 pour utilisation comme agent de lutte biologique.
